# FASCICULE DE BREVET EUROPEEN

(11) **EP 1 141 006 B1**
(45) Date de publication et mention de la délivrance du brevet: **28.09.2005**
(21) Numéro de dépôt: 99961115.5
(22) Date de dépôt: 20.12.1999
(51) Int. Cl.: C07K 14/47, C12N 15/52, C12N 15/90, A61K 38/17

(54) **INHIBITEUR DE LA TOPOISOMERASE II**
TOPOISOMERASE-II INHIBITOREN
TOPOISOMERASE II INHIBITOR

(30) Priorité: 22.12.1998 FR 9816264
(43) Date de publication de la demande: 10.10.2001
(73) Titulaire: LABORATOIRE L. LAFON, 94701 Maisons Alfort (FR)
(72) Inventeur: FERMANDJIAN, Serge, F-92190 Meudon (FR); FRERE-GALLOIS, Valérie, F-94200 Ivry-S/Seine (FR); TROALEN, Frédéric, F-92140 Bourg-la-Reine (FR)
(74) Mandataire: Bernasconi, Jean Raymond
(86) Numéro de dépôt international: PCT/FR1999/003208
(87) Numéro de publication internationale: WO 2000/037499

(56) Documents cités:
- FRERE-GALLOIS V ET AL.,: "Peptide fragments of DNA topoisomerase II with helix-forming and coiled-coil-forming properties act as inhibitors of the enzyme" EUROPEAN JOURNAL OF BIOCHEMISTRY, vol. 249 (1), 1997, page 142-148 XP002121994 cité dans la demande
- GUDKOV A.V. ET AL.,: "Isolation of genetic suppressor elements, inducing resistance to topoisomerase II-interactive cytotoxic drugs, from human topoisomerase II cDNA" PROCEEDINGS OF HE NATIONAL ACADEMY OF SCIENCES USA, vol. 90, 1993, pages 3231-3235, XP002121995
- KROLL D J: "Homologous and heterologous protein-protein interactions of human DNA topoisomerase II-alpha" ARCHIVES OF BIOCHEMISTRY AND BIOPHYSICS, vol. 345 (2), 1997, page 175-184 XP002121996
- GRIGOLO B ET AL.,: "Mapping of topoisomerase II alpha epitopes recognized by autoantibodies in idiopathic pulmonary fibrosis" CLINICAL AND EXPERIMENTAL IMMUNOLOGY, vol. 114 (3), décembre 1998 (1998-12), page 339-346 XP002121997
- BIERSACK H ET AL.,: "DNA topoisomerase II-alpha/beta heterodimers might serve as a new target for topoisomerase targeting drugs" FASEB JOURNAL, vol. 11 (9), 1997, page pA1034 XP002121998
- LANG A J ET AL: "Structural organization of the human TOP2A and TOP2B genes" GENE: AN INTERNATIONAL JOURNAL ON GENES AND GENOMES, vol. 221, no. 2, 23 octobre 1998 (1998-10-23), page 255-266 XP004143168 ISSN: 0378-1119

### LISTE DE SEQUENCES

<110> Laboratoire L. LAFON
<120> Inhibiteur de la topoisomérase II
<130> BET 99/1062
<140>
   <141>
<150> FR 9816264
   <151> 1998-12-22
<160> 2
<170> PatentIn Ver. 2.1
<210> 1
   <211> 29
   <212> PRT
   <213> Séquence artificielle
<220>
   <223> Description de la séquence artificielle:peptide synthétique inhibiteur de la topoisomérase II
   Xaa en position 2 = Gln ou Glu
   Xaa en position 6 = Leu ou Lys
   Xaa en position 7 = Arg ou Lys
   Xaa en position 10 = Phe ou Tyr
   Xaa en position 12 = Leu ou Lys
   Xaa en position 18 = Gly ou Glu
   Xaa en position 19 = Leu ou Glu
   Xaa en position 25 = Leu ou Glu
   Xaa en position 26 = Gln ou Lys
   Xaa en position 27 = Met ou Nle
<400> 1
   Thr Xaa Leu Asp Ile Xaa Xaa Asp Leu Xaa Glu Xaa Arg Leu Lys Tyr
    1               5                   10                  15
   Tyr Xaa Xaa Arg Lys Glu Phe Leu Xaa Xaa Xaa Leu Gly
               20                   25
<210> 2
   <211> 29
   <212> PRT
   <213> Séquence artificielle
<220>
   <223> Description de la séquence artificielle:peptide synthétique inhibiteur de la topoisomérase II
<400> 2
   Thr Gln Leu Asp Ile Leu Arg Asp Leu Phe Glu Leu Arg Leu Lys Tyr
    1               5                   10                  15
   Tyr Gly Leu Arg Lys Glu Phe Leu Leu Gln Met Leu Gly
               20                   25

## Description

La présente invention concerne un peptide présentant une forte activité inhibitrice de la topoisomérase Il humaine α et qui trouve une application en thérapeutique notamment comme antitumoraux.

L'ADN topoisomérase Il est une enzyme essentielle à la vie des cellules eucaryotes. Elle change la topologie de l'ADN par coupure transitoire double-brin d'une hélice d'ADN à travers laquelle elle fait passer une autre hélice d'ADN. A l'intérêt biologique de cette enzyme, s'ajoute un intérêt pharmacologique puisqu'elle est la cible privilégiée de nombreux agents antitumoraux (Corbett et Osheroff, Chem. Res. Toxicol., 6, 585, 1993).

L'un des problèmes majeurs de la chimiothérapie anticancéreuse actuelle est constitué par le manque de spécificité des agents antitumoraux ciblant la topoisomérase II. Afin de résoudre ce problème, nous avons développé une nouvelle classe d'inhibiteurs peptidiques de la topoisomérase Il intéragissant sélectivement avec cette enzyme et bloquant son activité catalytique.

Frère Gallois et al. ont décrit, dans Eur. J. Biochem., 249, 142, 1997, un fragment de la topoisomérase II correspondant à la séquence 1013 à 1041 de la topoisomérase II humaine a qui présente une activité inhibitrice de la topoisomérase II.

La présente invention vise à fournir des peptides qui présentent une activité inhibitrice supérieure.

La présente invention a ainsi pour objet un peptide présentant la séquence d'aminoacides suivante : dans laquelle :
X₁ = Gln
X₂ = Leu
X₃ = Arg
X₄ = Phe
X₅ = Leu
X₆ = Gly
X₇ = Leu
X₈ = Leu
X₉ = Gln
X₁₀ = Met
et ses dérivés protégés pharmaceutiquement acceptables.

Par dérivé protégé pharmaceutiquement acceptable, on entend en particulier des dérivés comprenant des groupes N-protecteurs pharmaceutiquement acceptables.

Des groupes N-protecteurs pharmaceutiquement acceptables sont notamment les groupes protecteurs de l'attaque en N-terminal des enzymes exopeptidases. Comme exemples de tels groupes on peut citer les groupes acyles tels que les groupes t-butyloxycarbonyle (Boc), tert-amyl-oxycarbonyle (⁺ Aoc), benzyloxycarbonyle, benzoyle, acétyle, formyle, propanoyle, butanoyle, phénylacétyle, phénylpropanoyle, cyclo pentylcarbonyle.

Les peptides selon l'invention peuvent être préparés de manière classique par synthèse peptidique en phase liquide ou solide par couplages successifs des différents résidus d'acides aminés à incorporer (de l'extrémité N-terminale vers l'extrémité C-terminale en phase liquide, ou de l'extrémité C-terminale vers l'extrémité N-terminale en phase solide) et dont les extrémités N-terminales et les chaînes latérales réactives sont préalablement bloquées.

Comme exemple de groupes bloquant les extrémités N-terminales, on peut citer : Boc, Bpoc, Fmoc.

La présente invention a également pour objet une composition pharmaceutique comprenant, à titre de principe actif, un peptide selon l'invention.

De telles compositions sont utilisables comme agent antiviral, antibactérien, antiparasitaire ou anticancéreux comme par exemple dans le traitement de tumeurs, de maladies parasitaires telles que la leishmaniose ou la maladie de Chagas ou bien dans le traitement des maladies des voies respiratoires telles que les otites ou pneumonies.

La vectorisation préalable du peptide peut s'avérer avantageuse dans la mesure où elle peut favoriser le contact entre le peptide et les cellules ciblées. Les méthodes de vectorisation sont connues de l'homme du métier. L'une de ces méthodes consiste à encapsuler le peptide dans des liposomes tels que des liposomes cationiques. On sait que ces liposomes assurent une protection supplémentaire du peptide vis-à-vis de la dégradation in vivo. La libération lente du principe actif au niveau des zones ciblées est donc ainsi très nettement favorisée. La première étape du processus de transfection des cellules ciblées par le peptide ou du processus de libération du peptide implique la formation d'un complexe entre la paroi lipidique du liposome et le peptide.

Après fusion des membranes respectives du liposome et de la cellule, il y a libération du peptide dans le cytoplasme.

La préparation de tels liposomes est décrite de façon détaillée dans "Liposome technology", Gregoriadis (CFC Press, NY 1984) ou encore dans "Liposomes", Ostro (Macel Dekker, 1987) ou bien encore dans la publication de Lichtenberg et al. parue dans "Methods Biochem. Anal. 33 : 337-462, 1988). Dans les liposomes, le peptide est soit dispersé, soit contenu, soit autrement emprisonné dans des corpuscules constitués de couches aqueuses concentriques adhérant à des couches lipidiques. Le peptide est soit contenu dans la phase aqueuse, soit contenu dans la phase lipidique et éventuellement dans chacune des deux phases en fonction de sa solubilité. La phase lipidique peut comprendre notament des phospholipides tels que la lécithine ou la sphingomycéline, des stéroïdes tels que le cholestérol, des tensioactifs tels que le dicétylphosphate, la stéarylamine, l'acide phosphatidique et/ou d'autres matériaux hydrophobes. Le diamètre des liposomes varie de préférence entre 15 nm et 5 µm.

On peut également envisager la vectorisation par incorporation du peptide dans les microsphères biodégradables.

La préparation des compositions de l'invention est réalisée de façon classique et dépend du mode d'administration envisagé.

Selon un mode de réalisation préféré de l'invention, les peptides éventuellement incorporés à un vecteur sont administrés par voie transdermique, transmuqueuse ou intratumorale. Les compositions de l'invention se présentent préférablement sous la forme de solutions ou suspensions injectables.

Comme véhicule pharmaceutiquement acceptable, on utilisera essentiellement des tampons, une solution saline de tampon phosphate (PBS) ou toute autre solution pour peu qu'elle présente un pH physiologiquement acceptable.

On peut également incorporer le principe actif dans une matrice ou un véhicule sous la forme de gel hydraté, par exemple un gel à base de copolymère oxyde de propylène-oxyde d'éthylène liquide au-dessous de la température ambiante et gélatineux à une température égale ou supérieure à la température ambiante. Avant administration, le gel peut être éventuellement fluidifié.

De façon à permettre une libération retardée du principe actif, on peut en variante formuler le peptide dans une composition à libération prolongée. De telles compositions ont été décrites dans la littérature. A cette fin, on peut notamment combiner le peptide à des polymères de l'acide polylactique.

Toutes ces compositions peuvent être appliquées localement par injection directe ou bien libérées à partir d'implants ou bien encore diffusées localement à l'aide d'une pompe appropriée.

La présente invention décrit également un procédé de traitement d'un patient présentant une tumeur, qui comprend l'administration à ce patient d'une quantité efficace d'un peptide selon l'invention.

La quantité de peptide à administrer dépend de la pathologie cancéreuse du sujet à traiter, de la gravité (présence ou non de métastases) de la maladie, du protocole de chimiothérapie sélectionné, du choix d'un traitement de première ou seconde intention et de la durée du traitement, ainsi que des conditions générales du patient. Les doses journalières peuvent être comprises entre 0,1 mg/kg et 10 mg/kg par jour ou par semaine selon le nombre de cycles de traitement de chaque cure.

### Exemple 1

### Peptide ayant la séquence

La synthèse du peptide correspondant a été réalisée par voie chimique en phase solide selon la stratégie Fmoc (9-fluorénylméthoxycarbonyl) sur un synthétiseur automatique Applied Biosystem 432 A en utilisant une résine HMP (4-hydroxyméthyl-phénoxyméthyl-copolystyrène-1% divinylbenzène) et l'activation par l'HBTU (2-(1H-benzotriazol-1-yl)-1,1,3,3-tétraéthyluronium hexafluorophosphate). Les extrémités N- et C- terminales ont été laissées libres. La déprotection a été faite par le TFA (acide trifluoroacétique). Le peptide déprotégé est ensuite filtré pour le séparer de la résine et précipité dans l'éther froid). Le peptide a été purifié par HPLC avec un appareillage Beckman System Gold en phase inverse sur une colonne Spherisorb C₁₈ (25x1 cm, 2 µm et 300 Å) avec un gradient linéaire de 0 à 100% d'acétonitrile/eau (avec 0,1% de TFA) sur 45 minutes, le débit étant de 4 ml/min. Les fractions présentant simultanément des pics d'absorption maximaux à 220 et 274 nm sont par la suite caractérisées en HPLC analytique et les fractions contenant le peptide pur sont groupées et lyophilisées.

Le peptide après purification a été caractérisé par :
- une HPLC analytique en phase inverse avec une colonne C₁₈ Nucleosyl (25x0,46 cm, 5 µm et 300 Å) en utilisant un gradient acétonitrile/eau (avec 0,1% de TFA) sur 45 minutes avec un débit de 1 ml/min.;
- une analyse de la composition en acides aminés avec un appareil Beckman AAA-6300;
- un séquençage sur un appareil Applied Biosystem 477 A utilisant la méthode de dégradation d'Edman;
- une spectroscopie de masse (ESIMS) sur un instrument quadrupole VG Biotech.

### INHIBITION DE LA TOPOISOMERASE II

### Test d'activité

Deux tests enzymatiques permettent d'estimer l'effet des peptides sur l'activité de la topoisomérase II : la relaxation de plasmides surenroulés et la décaténation d'ADN de kinétoplastes (Wang, J.C., An. Rev. Biochem., 54 : 665-697,1985 ; Maxwell, A., Advances in Protein Chemistry, 38 : 69-107, 1986 ; Liu, L.F., Cell, 19 : 697-707, 1980). Le peptide de l'exemple 1 inhibe totalement l'activité de la topoisomérase II humaine α et celle de levure dans les deux tests à une concentration de 0,5 µM.

## Revendications

1. Peptide présentant la séquence d'aminoacides suivante :

2. Composition thérapeutique comprenant, à titre de principe actif, un peptide selon la revendication 1.

3. Composition thérapeutique ayant une activité inhibitrice de la topoisomérase II humaine α comprenant, à titre de principe actif, un peptide selon la revendication 1.

4. Utilisation, pour la fabrication d'une composition thérapeutique ayant une activité inhibitrice de la topoisomérase II humaine α, d'un peptide selon la revendication 1.

## Patentansprüche

1. Peptid, das die folgende Aminsosäuresequenz aufweist:

2. Therapeutische Zusammensetzung, die als aktive Basis ein Peptid nach Anspruch 1 aufweist.

3. Therapeutische Zusammensetzung, die eine inhibitierende Wirkung auf menschliche Topoisomerase II aufweist, umfassend als wirksame Basis ein Peptid nach Anspruch 1.

4. Verwendung eines Peptids nach Anspruch 1 zur Herstellung einer therapeutischen Zusammensetzung mit einer inhibitierenden Aktivität auf die menschliche Topoisomerase II.

## Claims

1. Peptide having the following amino acid sequence:

2. Therapeutic composition comprising, as an active principle, a peptide according to Claim 1.

3. Therapeutic composition having inhibitory activity for human topoisomerase II alpha, comprising, as an active principle, a peptide according to Claim 1.

4. Use, for manufacturing a therapeutic composition having inhibitory activity for human topoisomerase II alpha, of a peptide according to Claim 1.
